# EUROPEAN PATENT SPECIFICATION

(11) **EP 1 648 541 B1**
(45) Date of publication and mention of the grant of the patent: **08.09.2010**
(21) Application number: 04779216.3
(22) Date of filing: 27.07.2004
(51) Int. Cl.: A61M 5/50, A61M 5/315

(54) **SYRINGE ASSEMBLY HAVING DISABLING MECHANISM**
SPRITZVORRICHTUNG MIT EINER VERRIEGELUNGSVORRICHTUNG
ENSEMBLE SERINGUE A MECANISME DE DESACTIVATION

(30) Priority: 30.07.2003 US 490939 P; 04.05.2004 US 838687
(43) Date of publication of application: 26.04.2006
(73) Proprietor: Becton Dickinson and Company, Franklin Lakes, New Jersey 07417-1880 (US)
(72) Inventor: SAMUEL, Suresh, P., R., Coimbatore 42 (IN); SMITH, Chad, Oak Ridge, NJ 07438 (US); CAIZZA, Richard, James, Vermon, NJ 07462 (US)
(74) Representative: von Kreisler Selting Werner
(86) International application number: PCT/US2004/024045
(87) International publication number: WO 2005/011782

(56) References cited:
- WO-A-94/13336
- DE-U- 8 914 128
- FR-A- 2 646 087
- US-A- 5 106 372
- US-A- 5 222 942
- US-A- 6 083 200

## Description

### Background of the Invention

The present invention relates to syringe assemblies and particularly to syringe assemblies having an automatic disabling mechanism.

Throughout the world the multiple use of hypodermic syringe products which are intended for single-use only, is instrumental in drug abuse and in the transfer of contagious diseases. Intravenous drug users who routinely share and re-use syringes are a high-risk group with respect to the AIDS virus. Also, the effects of multiple use are a major concern in some countries where repeated use of syringe products during mass immunization programs may be responsible for the spread of many diseases. Re-use of single-use hypodermic syringe assemblies is also instrumental in the spread of drug abuse even in the absence of infection or disease.

Many attempts have been made to remedy this problem. Most notable are early contributions which relied on a specific act to destroy the syringe after use either by using a destructive device or providing a syringe assembly with frangible zones so that the syringe could be rendered inoperable by the application of force. Other attempts involve the inclusion of structure which would allow the destruction or defeating of the syringe function to a conscious act by the syringe user. Although many of these devices work quite well, they do require the specific intent of the user followed by the actual act to destroy or render the syringe inoperable. These devices are not effective with a user having the specific intent to re-use the hypodermic syringe. Accordingly, there was a need for a single-use hypodermic syringe which after use will become inoperable or incapable of further use automatically without any additional act on the part of the user. The automatic function is much harder to provide because the means for rendering the syringe inoperable must not prevent its filling or use under normal conditions.

A single-use syringe which automatically disables after injection is taught in U.S. Patent No. 4,973,310 to Kosinski. This syringe contains a locking element positioned in the syringe barrel between the plunger rod and the inside surface of the barrel. In use, the syringe allows the user to draw a pre-selected amount of medication into the chamber of the barrel and deliver this medication, as through injection, into the patient. Any attempt to withdraw the plunger to use the syringe a second time will cause the locking element to embed itself into the inside surface of the syringe barrel to prevent proximal motion of the plunger rod.
The first part of claim 1 refers to an operable syringe assembly as disclosed in DE 89 14 128 U. This one way injection syringe comprises an elongate hollow plunger rod forming a hollow plunger portion comprising a H-shaped component with two spring tongues extending from either side of a base portion in opposite directions. The spring tongues engage engagement rings of the hollow plunger portion. The component is connected with a plunger plate through a plunger rod. When the syringe assembly is used for drawing liquid into the syringe barrel, the spring arms of the H-shaped component are disengaged from the ring portions of the hollow plunger rod so that they move inwardly and the H-shaped component is released from the plunger rod. This allows only one drawing-in operation of the syringe. Thereafter, the H-shaped component is on its both ends released from the hollow plunger rod so that the plunger plate is no longer connected with the plunger rod.
WO 94/13336 discloses a non-reusable syringe with a needle port. The syringe comprises a piston releasably connected to a plunger so that it is rendered inoperable after a single use. The piston has a plurality of rearwardly projecting latch arms engageable with a retaining ring provided on the forward end of the plunger. A retaining collar is provided for locking the latch arms in the engagement position. When liquid is withdrawn into the syringe barrel, the retaining collar remains in the locking position. Thereafter, the liquid is expelled from the syringe barrel, whereby the latch arms become released from the retaining collar so that the piston is no longer connected with the plunger rod.
U.S. 5,222,942 A describes a syringe having a locking means associated with a plunger or plunger rod, which may be used to administer the maximum volume of the syringe only once. The plunger rod has a serration of saw-tooth design cooperation with a stopping collar. The action of the stopping collar produces the consequence that even though the plunger rod may be pushed into the cylinder, a repeated withdrawal in the direction of displacement is no longer possible since this withdrawal is prevented by catches bearing against a tooth flank. Thus, an idle stroke is still possible, but a repeated stroke for aspiration is prevented. These provisions ensure that a syringe is usable for an intended single application.

There is still a need for a single-use syringe which will allow a pre-selected number of plunger rod strokes before the automatic disabling mechanism activates. For example, four strokes of the plunger may be required to complete the injection process. Such as when the syringe assembly is used to draw a diluent into the syringe barrel dispense the diluent into a vial containing the substances to be reconstituted, drawing back the reconstituted medication into the syringe and then delivering the contents of the syringe into the patient.

### Summary of the Invention

It is an object of the invention to provide an operable syringe assembly having a passive disabling structure.

The syringe assembly of the present invention is defined by claim 1.

An operable syringe assembly having a passive disabling structure includes a barrel having a cylindrical sidewall with an inside surface defining a chamber for retaining fluid, an open proximal end and a distal end including a distal wall having a passageway therethrough in fluid communication with the chamber. An elongate hollow plunger rod having a proximal end, an open distal end, and an interior surface is provided. A stopper has a circular-shaped sealing element having a peripheral surface forming a seal with the inside surface of the barrel and a boss member projecting proximally from the sealing element. A locking element interacts between the stopper and the plunger rod. The locking element includes a central body portion having at least one cantilevered leg extending distally outwardly from the body portion wherein the leg includes a sharp free end directed outwardly for engaging the inside surface of the barrel. The locking element is movably connected to the boss of the stopper and movably connected to the plunger rod interior surface. Structure for indexing the locking element distally in the plunger rod during proximal motion of the plunger rod to draw fluid into the chamber and for indexing the locking element distally on the boss of the stopper during distally-directed motion of the plunger rod for delivering fluid from the chamber through the passageway and means for engaging the locking element with the inside surface of the barrel sidewall for preventing reuse of the syringe assembly is provided.

Structure for engaging the locking element with the inside surface of the barrel sidewall may include an opening in the distal end of the plunger rod to shorten the axial length of the interior surface area of the opening for allowing the sharp free end to project outwardly past the plunger rod and onto the inside surface of the barrel.

The structure for indexing includes at least one detent on the interior surface of the distal end of the plunger rod, at least one boss detent on the boss and at least one cantilevered arm on the stopper having an outwardly extending rib near its free end sized to engage a recess in the inside surface of the plunger rod. The indexing structure further includes the locking element having at least one finger element extending inwardly from an aperture in the central body portion of the locking element. The locking element is positioned with its sharp free end contacting the interior surface of the plunger rod proximally of the at least one detent and the boss member is positioned in the aperture of the locking element wherein the at least one finger element is contacting the boss member proximally of the at least one boss detent and the outwardly extending rib is positioned in the recess of the plunger rod.

The syringe assembly may be configured so that the at least one detent in the plunger rod includes two axially-spaced detents and the at least one detent in the boss includes two axially-spaced boss detents so that the plunger rod can be moved distally one or two times before proximal motion of the plunger rod causes the locking element to engage the inside surface of the barrel, depending on the initial position of the locking element at the time of use. The two axially-spaced detents in the plunger rod may include two axially-spaced steps each having a blunt surface at its distal end extending inwardly from the interior surface of the plunger rod. The two axially-spaced boss detents may each include an inclined surface extending proximally inwardly and a blunt surface at a distal end of each of the inclined surfaces extending radially inwardly.

The at least one cantilevered leg of the locking element may include two cantilevered legs positioned on opposite sides of the central body portion. The stopper may further include two radial cam projections positioned to contact and force the two cantilevered legs outwardly when excessive proximally-directed force is applied to the plunger rod in an attempt to overcome the locking element's engagement to the inside surface of the barrel.

The at least one cantilevered arm of the stopper may include two cantilever arms positioned on opposite sides of the boss and said at least one recess in the inner surface of the plunger rod may include two recesses positioned on opposite sides of the plunger rod and configured to receive the outwardly extending ribs of the two cantilevered arms.

The syringe barrel may further include an elongate tip extending distally from the distal wall and having a passageway therethrough in fluid communication with the chamber of the syringe barrel. The syringe assembly may also include a needle cannula having a distal end, a proximal end and a lumen therethrough, wherein the proximal end of the needle cannula is connected to the distal end of the syringe barrel so that the lumen is in fluid communication with the passageway of the barrel. The syringe assembly may include a locking element made of sheet metal such as stainless steel. Further, the stopper and all its elements may be integrally formed of thermoplastic material.

### Brief Description of the Drawings

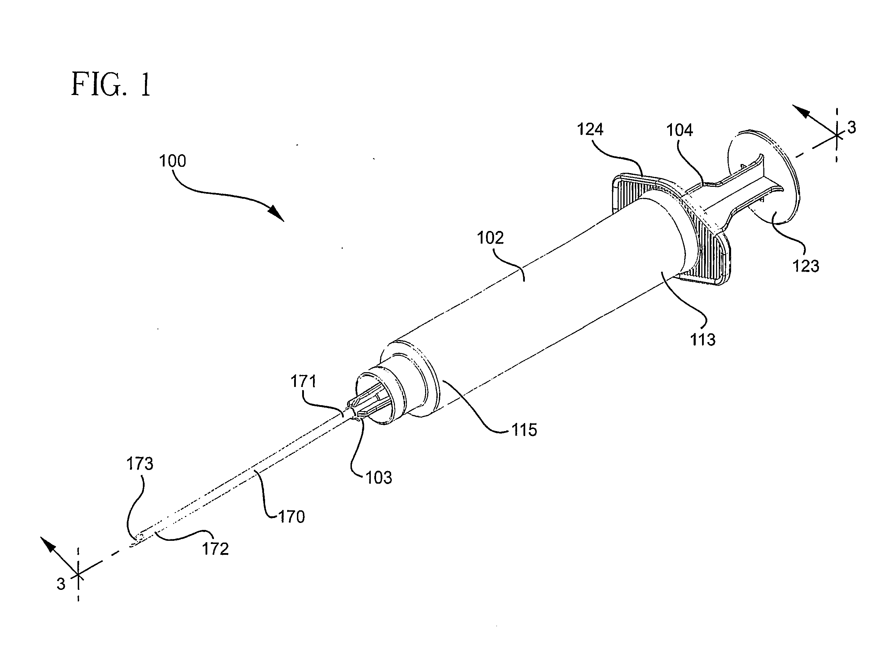

Fig. 1 is a side-elevational view of the syringe assembly of the present invention.

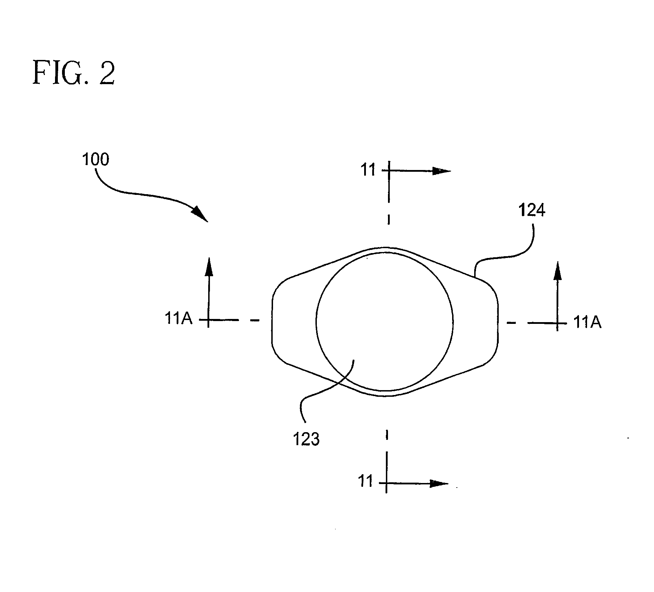

Fig. 2 is a side-elevational end view of the proximal end of the syringe assembly of Fig. 1.

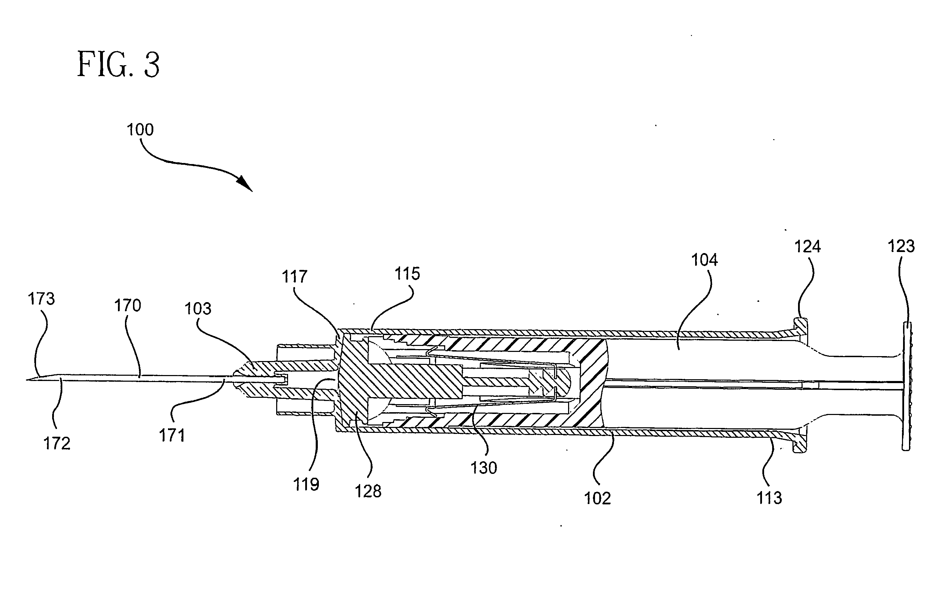

Fig. 3 is a cross-sectional view of the syringe assembly of Fig. 1 taken along line 3-3.

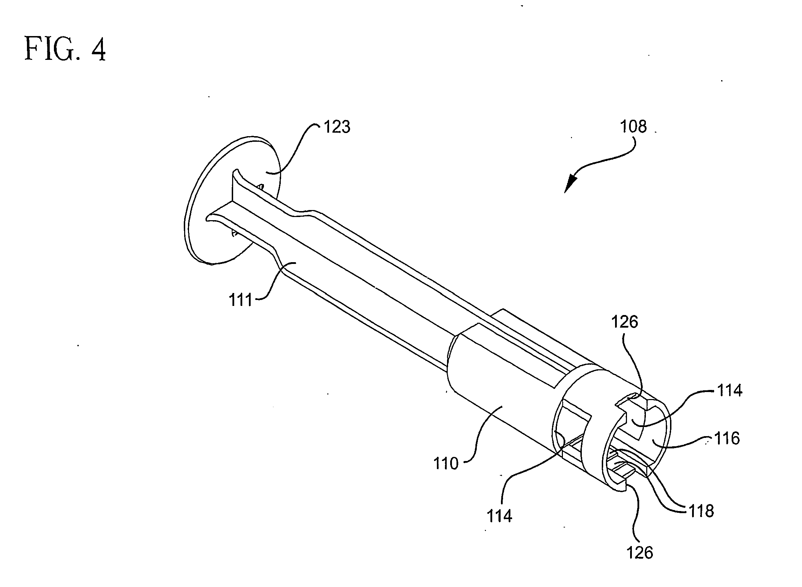

Fig. 4 is a perspective view of the plunger rod of the syringe assembly viewed from its distal end.

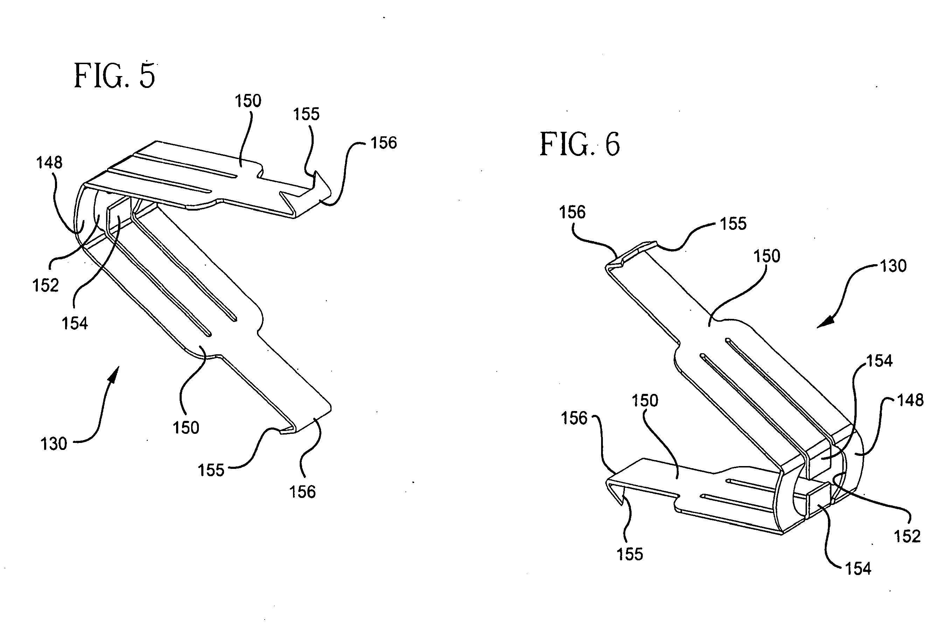

Fig. 5 is a perspective view of the locking element of the syringe assembly viewed from its distal end.

Fig. 6 is a perspective view of the locking clip viewed from its proximal end.

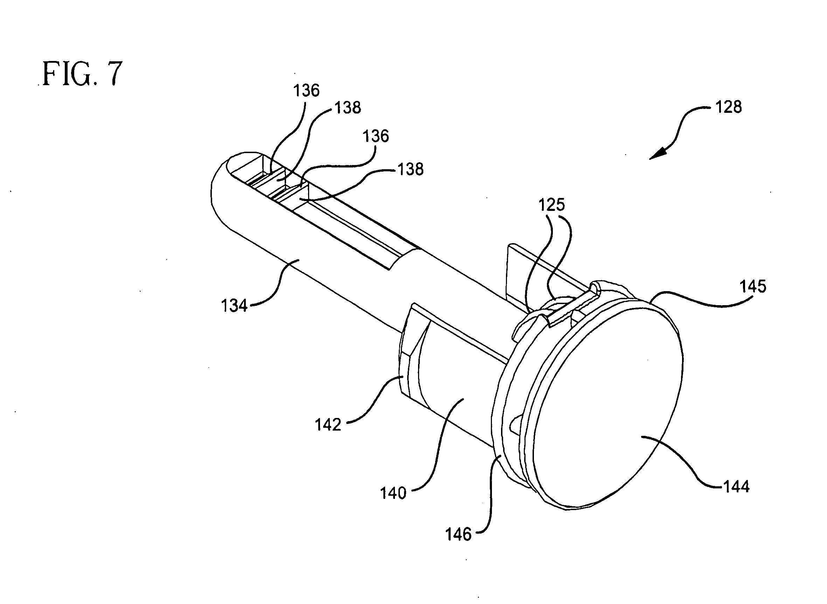

Fig. 7 is a perspective view of the stopper of the syringe assembly viewed from its distal end.

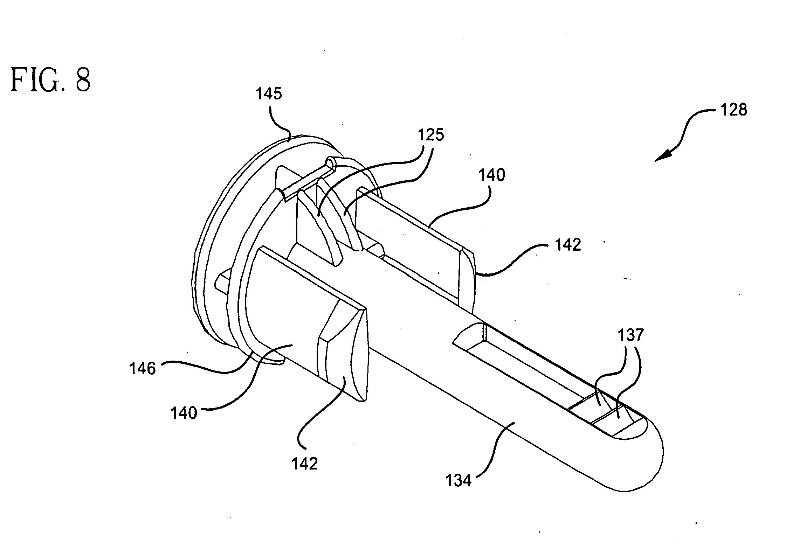

Fig. 8 is a perspective view of the stopper viewed from its proximal end.

Fig. 9 is an exploded side-elevational view of the plunger assembly.

Fig. 10 is a cross-sectional view of the plunger assembly of Fig. 9 taken along line 10-10.

Fig. 11 is an enlarged partial cross-sectional view of the syringe assembly of Fig. 1 taken along line 3-3 showing the syringe assembly before use.

Fig. 11A is an enlarged partial cross-sectional view similar to Fig. 11 but rotated 90°.

Fig. 12 is an enlarged partial cross-sectional view of the syringe assembly of Fig. 1 taken along line 3-3 showing the syringe assembly after a first aspiration stroke.

Fig. 12A is an enlarged partial cross-sectional view similar to Fig. 12 but rotated 90°.

Fig. 13 is an enlarged partial cross-sectional view of the syringe assembly of Fig. 1 taken along line 3-3 showing the syringe assembly during a first dispensing stroke.

Fig. 13A is an enlarged partial cross-sectional view similar to Fig. 13 but rotated 90°.

Fig. 14 is an enlarged partial cross-sectional view of the syringe assembly of Fig. 1 taken along line 3-3 showing the syringe assembly at the start of a second aspiration stroke.

Fig. 14A is an enlarged partial cross-sectional view similar to Fig. 14 but rotated 90°.

Fig. 15 is an enlarged partial cross-sectional view of the syringe assembly of Fig. 1 taken along line 3-3 showing the syringe assembly after a second dispensing stroke.

Fig. 15A is an enlarged partial cross-sectional view similar to Fig. 15 but rotated 90°.

Fig. 16 is an enlarged partial cross-sectional view of the syringe assembly of Fig. 1 taken along line 3-3 showing a position of the internal components in the event of an attempt to withdraw the plunger rod after the second dispensing stroke.

Fig. 16A is an enlarged partial cross-sectional view similar to Fig. 16 but rotated 90°.

Fig. 17 is an enlarged partial cross-sectional view similar to the syringe assembly of Fig. 16 showing the interaction of additional structure to prevent reuse.

Fig. 17A is an enlarged partial cross-sectional view similar to Fig. 17 but rotated 90°.

Fig. 18 is an enlarged partial cross-sectional view similar to the syringe assembly of Fig. 17 showing a discontinuity in the syringe barrel for engaging the locking element.

### Detailed Description of the Invention

The present invention is directed to a syringe assembly having a passive disabling mechanism. The disabling mechanism enables variable dosages by the syringe assembly and enables a selected number of cycles or strokes by the plunger rod before being automatically disabled. In one preferred embodiment, the disabling mechanism provides two aspirating and two dispensing cycles before being automatically disabled. The assembly enables the aspiration and dispensing of a selected volume of a diluent into a vial to reconstitute a drug, pharmaceutical agent, or other substance and then aspirating the reconstituted substance back into the syringe. A selected volume of the reconstituted substance can be injected or delivered to a patient where the volume of the substance that is delivered can be the same or different than the volume of the substance aspirated into the syringe barrel. The syringe is automatically disabled after the injection or delivery stroke by retracting the plunger rod, which activates the disabling mechanism.

The disabling mechanism is actuated by the axial movement of the plunger rod with respect to the syringe barrel and to the stopper, by moving the plunger rod in the aspirating direction. The stopper is coupled to the plunger rod to allow limited axial movement of the stopper with respect to the plunger rod. The disabling mechanism moves through a series of stages by reversing the direction of the axial movement of the plunger rod with respect to the stopper to move the mechanism in a step-wise manner to the disabling position. The disabling position of the mechanism is attained by the relative movement between the plunger rod and the stopper and is not dependent on the position of the stopper within the syringe barrel or the length of the stroke by the stopper. In this manner, the syringe assembly is able to dispense a desired volume of the drug or other substance, and the disabling mechanism can be actuated after the final dispensing or injection stroke regardless of the position of the stopper in the syringe barrel. By actuating the disabling mechanism, the stopper cannot be retracted to aspirate a substance into the syringe barrel but allows any substance remaining in the syringe barrel to be dispensed.

Referring to the drawings, a syringe assembly **100** having a disabling mechanism includes a syringe barrel **102** and a plunger assembly **104.** Barrel **102** includes a cylindrical sidewall **106** having an inside surface **107** defining a chamber **109** for retaining fluid, an open proximal end **113** and a distal end **115** including a distal wall **117** having a passageway **119** therethrough in fluid communication with the chamber. In this embodiment, the distal wall of the barrel includes an elongate tip extending distally therefrom and having a passageway in fluid communication with the passageway in the distal wall. In this embodiment barrel **102** also includes a needle cannula **170** having a proximal end **171,** a distal end **172** and a lumen **173** therethrough. The proximal end of the needle cannula is attached to elongate tip **103** so that the lumen of the needle cannula is in fluid communication with passageway **119** in the barrel.

Plunger assembly **104** includes an elongate hollow plunger rod **108,** a stopper **128** and a locking element **130.** Plunger rod **108** includes a proximal end **111,** an open distal end **110** and an interior surface **116** and at least one aperture or recess **114** in the interior surface at the distal end of the plunger rod. The recess includes a distal face **121.** In this embodiment, there are two recesses **114** having distal faces **121.** The interior surface at the distal end of the plunger rod includes at least one detent. In this embodiment the at least one detent on the interior surface of the distal end of the plunger rod includes four axially spaced detents **118** with two detents on each side of the plunger rod. Each pair of detents is shaped to form axially spaced steps **120** with each step having a blunt surface **122** at its distal end extending inwardly from the interior surface of the plunger rod.

Stopper **128** includes a circularly-shaped sealing element **144** having a peripheral surface **145** forming a seal with the inside surface of the barrel. A boss member **134** extends proximally from the sealing element and includes at least one boss detent and in this embodiment, contains two boss detents **136.** At least one cantilevered arm extends proximally from the sealing element and in this embodiment there are two cantilever arms **140** extending proximally from the sealing element. Each of the cantilevered arms includes an outwardly extending rib **142.** The rib is sized to fit within recess **114** in the plunger rod. The axially spaced boss detents **136** each include an incline surface **137** extending proximally inwardly and a blunt surface **138** at the distal end of each of the inclined surfaces. The stopper is preferably integrally formed of thermoplastic material such as polyethylene. The circularly-shaped sealing element and/or the peripheral sealing surface thereon may be made of elastomeric materials such as thermoplastic elastomers, natural rubber, synthetic rubber and combinations thereof.

Locking element **130** includes a central body portion **148** having an aperture **152** therethrough and at least one cantilevered leg **150** extending distally outwardly from the body portion and at least one finger element **154** extending inwardly from the aperture. In this embodiment, at least two cantilevered legs with each of the cantilevered legs having a sharp free end **155** directed outwardly for engaging the inside surface of the barrel. The configuration of sharp free end **155** can be any configuration capable of engaging the inside surface of the barrel, such as a sharp edge or one or more pointed teeth and the like. The locking element may be made of a variety of materials, or combinations of materials, however, it is preferred to have the sharp free ends made of metal and it is also preferred that the entire locking element be made of integrally formed from sheet metal such as stainless steel.

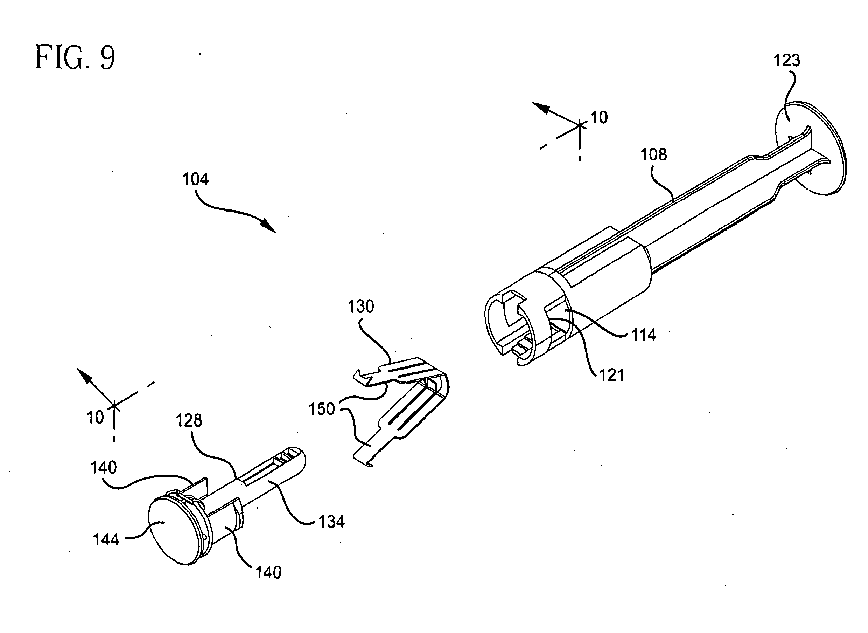

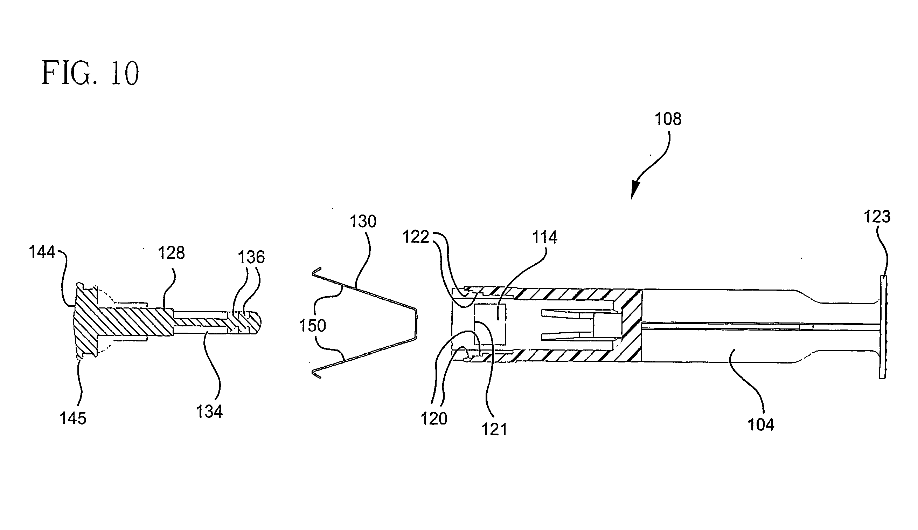

In this preferred embodiment plunger assembly **104** is assembled by inserting locking element **130** into the distal end of plunger rod **108.** Boss **134** of stopper **128** is then inserted into the distal end of the plunger rod through aperture **152** of locking element **130** so that cantilevered legs **150** extend toward circularly-shaped sealing element **144** of the stopper as illustrated in Figs. 9 and 10.

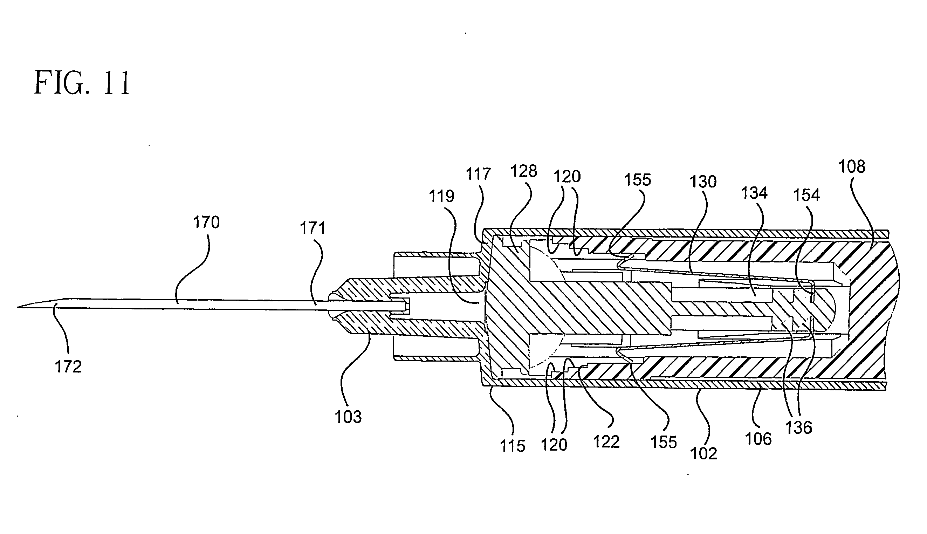

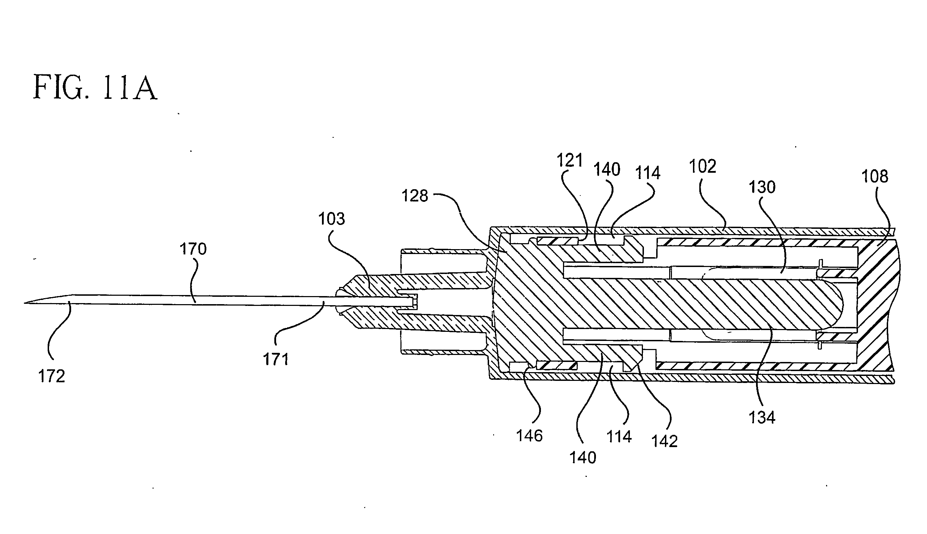

As will be explained in more detail hereinafter, the plunger assembly is then inserted into barrel **102** through open proximal end **113** to the initial position illustrated in Figs. 11 and 11A. In the initial position of the syringe element, locking element **130** is positioned with its sharp free ends **155** contacting the interior surface of the plunger rod proximally of axially spaced steps **120.** Boss member **134** is positioned in aperture **152** of locking element **130** so that finger elements **154** contact boss member proximally of boss detents **136.** Outwardly extending ribs **142** of cantilever arms **140** are positioned in recesses **114** in the plunger rod. Ribs **142** are configured to complement the recesses **114** for allowing limited axial movement of the stopper with respect to the plunger rod. The stopper **128** further includes stabilizing member **146** positioned proximally with respect to sealing element **144** and has an outer dimension complimenting the other dimension of the sealing element as shown in Fig. 11A, stabilizing member **146** has an outer dimension to contact the inner surface of the syringe barrel and is spaced from sealing element **144** to assist in stabilizing stopper **128** to maintain the stopper and boss member **134** in an orientation substantially parallel to the axis of the syringe barrel. In the position illustrated in Figs. 11 and 11A, syringe assembly **100** is ready to use for drawing liquid into the chamber of the barrel.

As will now be shown, the operation of the plunger assembly of this embodiment includes a first aspiration stroke followed by a first dispensing stroke, a second aspiration stroke and a final dispensing stroke after which the syringe is disabled. The disabling elements prevent or inhibit movement of stopper **128** in a proximal aspirating direction thereby limiting the function of the syringe assembly to a single use. The maximum number of strokes being limited by a number of axially positioned detents in the plunger rod and the number of axially positioned boss detents on the stopper. However, the actual number of strokes the syringe may make will be determined by the position of the locking element with respect to the detents in the plunger rod and the detents on the stopper at the time of first use. For example, a syringe with two plunger detents and two stopper detents can be supplied to the end user as a syringe capable of two strokes or four strokes. This is an important feature of the present invention since a single syringe assembly can be provided with different stroke limitations before disabling.

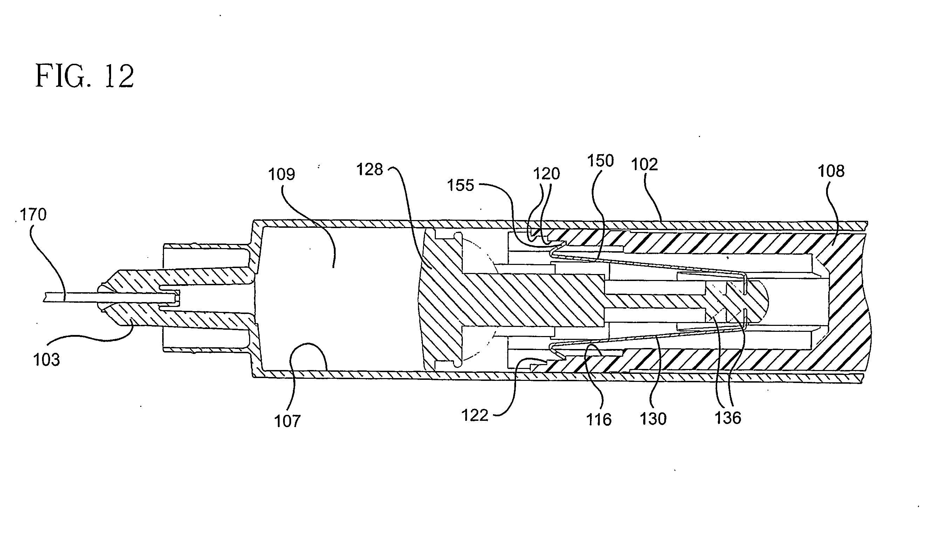

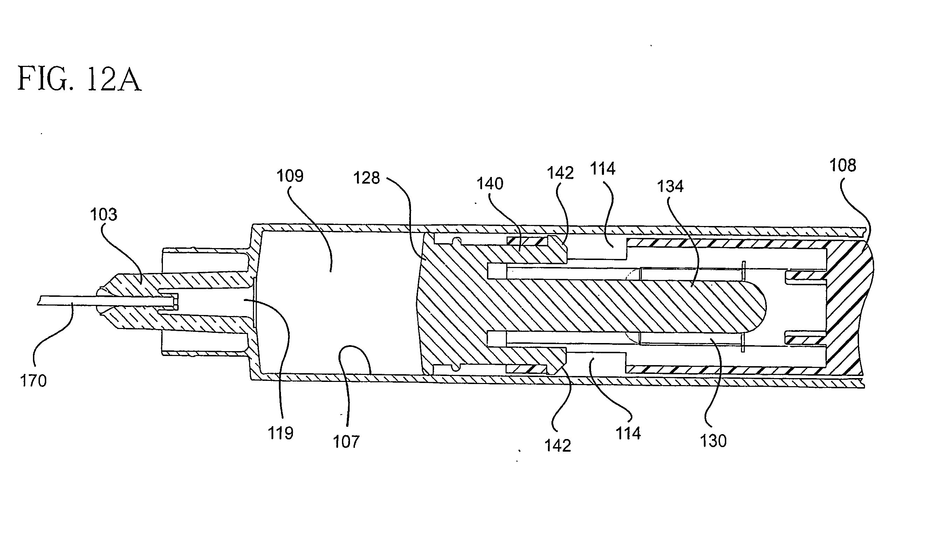

The syringe assembly may now be used to draw liquid, such as a sterile water diluent into the chamber of the barrel by applying a proximally directed force to a thumb press **123** on the proximal end of the plunger rod while holding the syringe barrel. As illustrated in Figs. 12 and 12A, this causes the plunger rod to move proximally with respect to the stopper until the free end of cantilevered legs **150** moves distally along inner surface **116** of the plunger rod and snaps past blunt surface **122** of the proximal most axially spaced steps **120,** as best illustrated in Fig. 12. Also, during this first aspiration stroke outwardly extending ribs **142** engage distal surface **121** of the recesses **114** in the plunger rod as best illustrated in Fig. 12A. When ribs **142** engage distal surface **121** the stopper is drawn proximally with respect to the barrel as the plunger rod moves. The stopper is now moved proximally, through action of the plunger rod, until the desired volume of liquid is in the chamber as determined by the user.

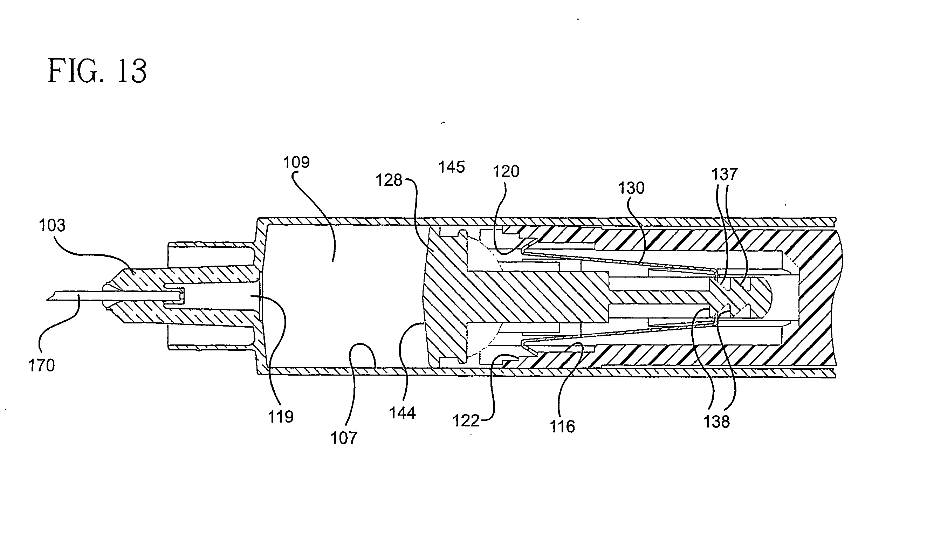

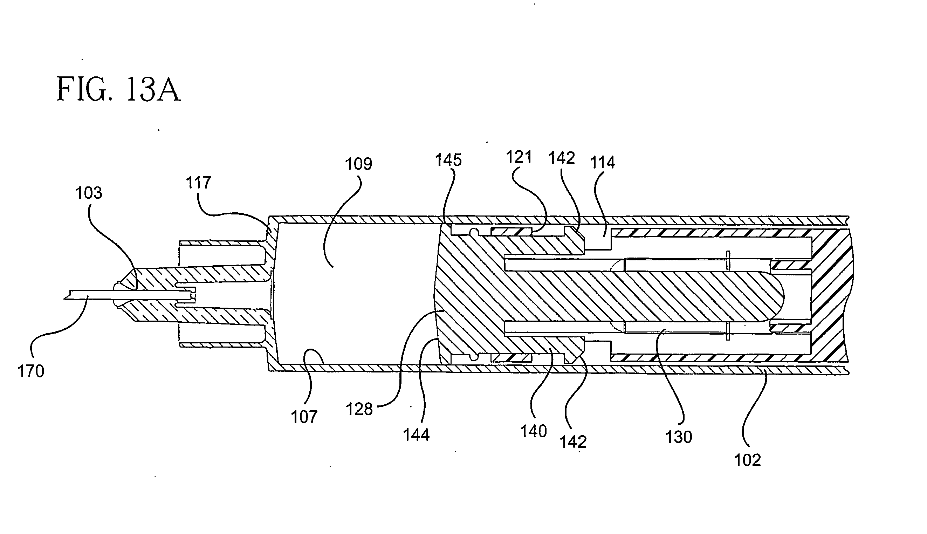

The liquid diluent in the chamber may now be discharged into a vial of dry medication such as lyophilized medication, for reconstitution. This first dispensing stroke is accomplished by moving the plunger rod in a distal direction while holding the barrel. A barrel flange **124** is provided on the proximal end of the barrel to help control motion of the barrel during use of the syringe assembly. As best illustrated in Figs. 13 and 13A, as the plunger rod moves distally, locking element **130** moves with the plunger rod dragging the locking element with it so that finger elements **154** on the locking element slide from the proximal most to the distal most boss detent by riding up inclined surface **137** and falling into the second detent. When the plunger rod contacts the stopper, the stopper will begin moving in a distal direction along with the plunger rod to discharge liquid diluent from the chamber into, for example, a vial of lyophilized medication.

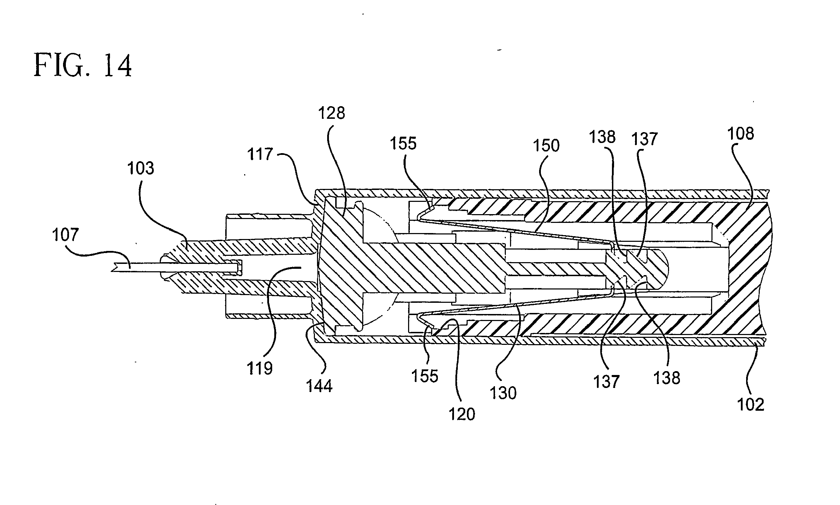

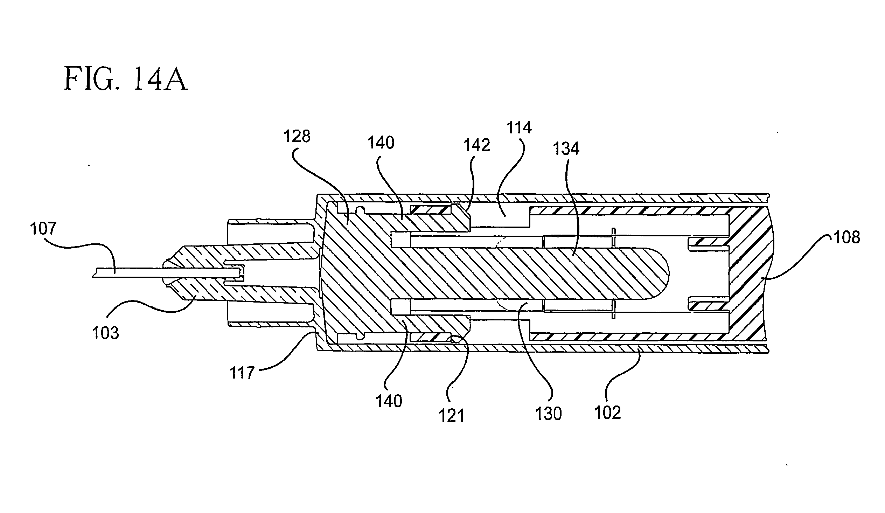

When the diluent and the lyophilized medication are mixed the syringe assembly of the present invention may now be used to withdraw the reconstituted, ready-to-inject medication into the chamber of the syringe barrel, as best illustrated in Figs. 14 and 14A, by applying a proximally directed force to the plunger rod while holding the syringe barrel. Proixmally directed force will cause the plunger rod to move in a proximal direction while locking element **130** will remain relatively stationary due to its connection to the boss detent on the stopper. Proximal motion of the plunger causes the locking element to move distally along the inside surface of the plunger rod so that the sharp free end **155** of the cantilever legs moves from the proximal-most axially-spaced steps **120** in the plunger rod to the second more distal axially-spaced steps **120.** Proximal motion of the plunger rod also causes outwardly extending ribs **142** of cantilever arms **140** to engage distal surfaces **121** of recesses **114** in the plunger rod so that the stopper now moves proximally with the plunger rod drawing the reconstituted medication into the chamber of the syringe barrel to an amount determined by the user. An advantage of the present invention is that the amount of medication drawn into the chamber, and therefore the maximum amount of medication that can be delivered, is determined by the user at the time of use and not by the placement of the components at the time of manufacture.

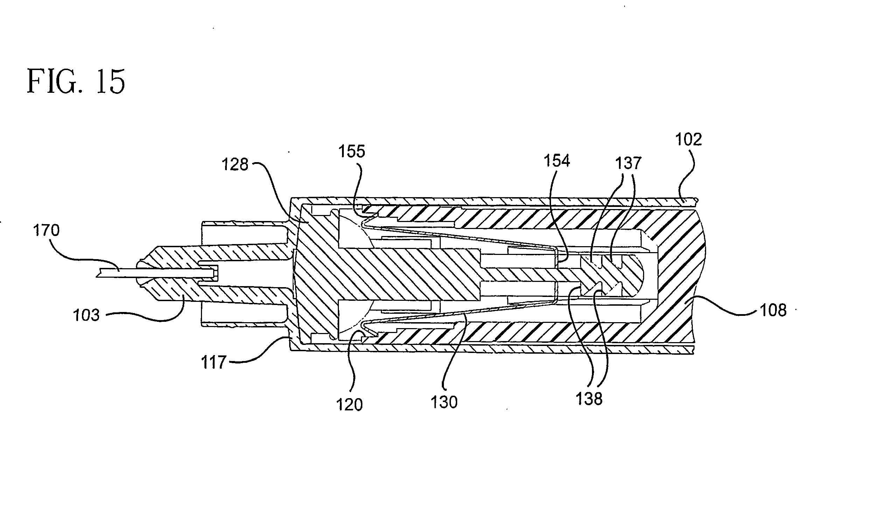

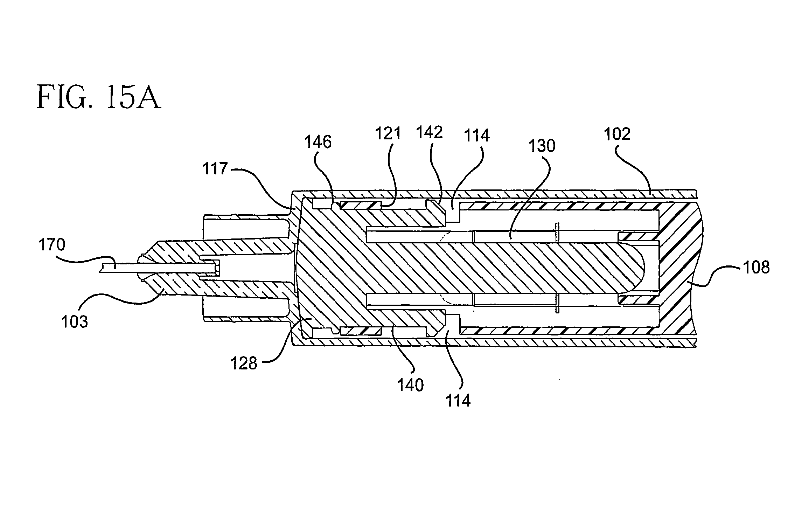

The syringe assembly of the present invention is now ready for a second and final dispensing stroke which is best illustrated in Figs. 15-15A. The medication is delivered to the patient by applying a distally directed force to the plunger rod causing the plunger rod to move in a distal direction with respect to the barrel. As the plunger rod advances in a distal direction the engagement of sharp free ends **155** of the locking element in with the distal-most blunt surfaces **122** of axially-spaced steps **120** moves the locking element distally so that finger elements **154** of the locking element ride over the distal-most inclined surface **137** of the boss detents distally past the most distal boss detent **136.** When the distally moving plunger rod contacts the stopper, both the stopper and the plunger rod move toward the distal end of the barrel to discharge the contents of the chamber through the passageway.

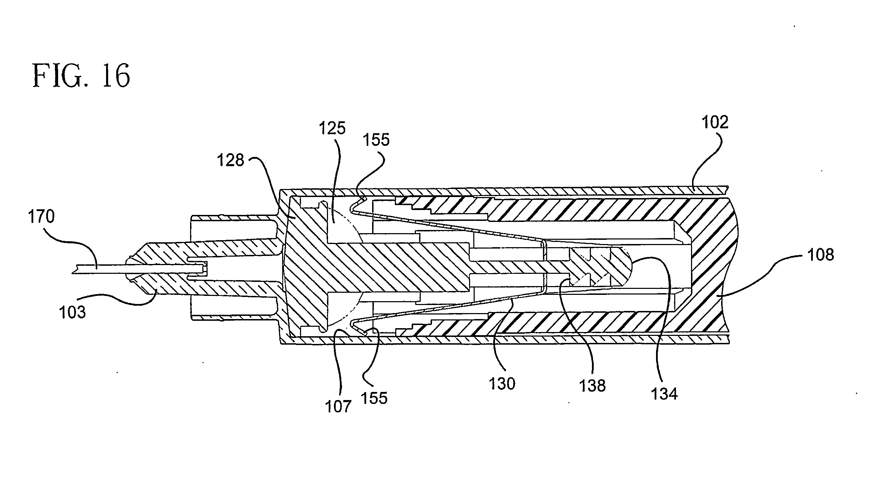

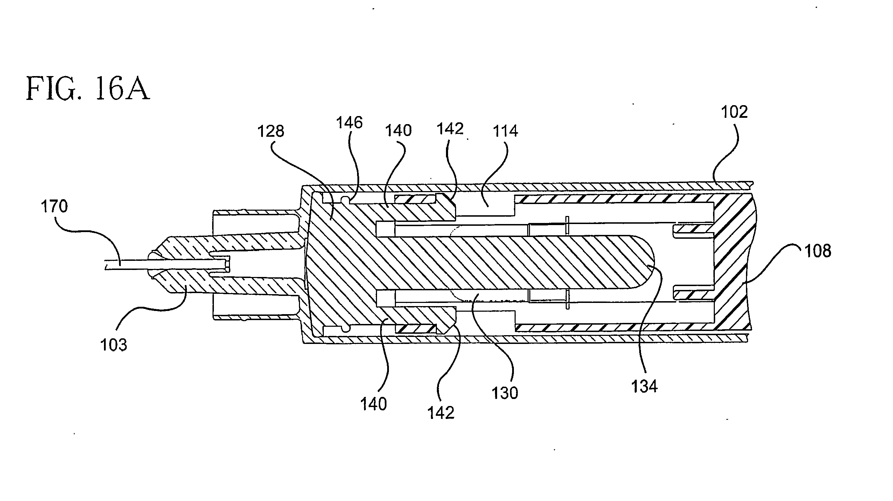

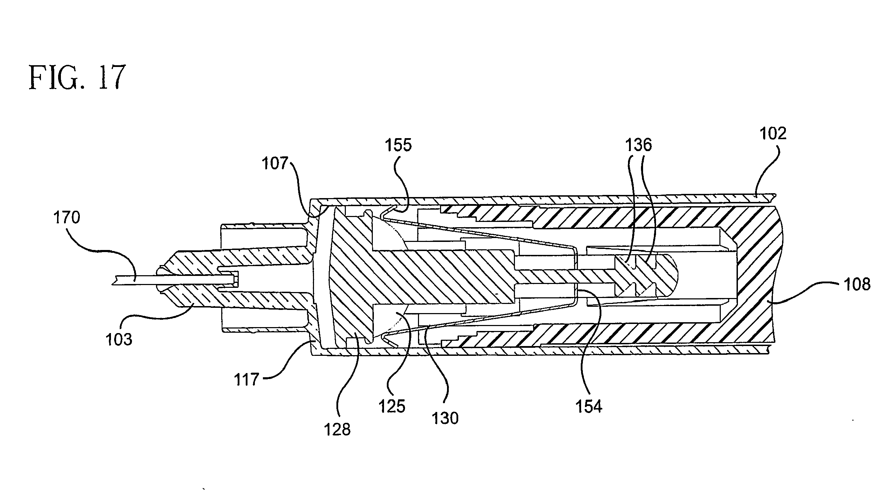

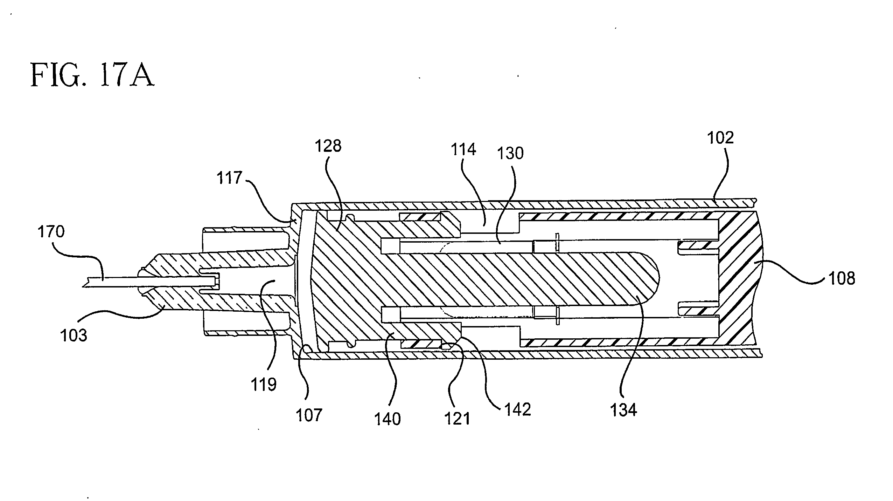

The syringe assembly has now been used and is ready to be discarded. Any attempt to move the plunger rod in a proximal direction to refill the syringe assembly for further use will cause the locking element to disable the syringe. Specifically, as best illustrated in Figs. 16 and 16A, moving the plunger rod in a proximal direction will allow the plunger rod to move a short distance until the sharp free ends **155** of the locking clip snap past the end of the plunger rod and engage the inside surface **107** of the barrel side wall. Further proximal motion of the plunger will be resisted by the locking element's engagement to the inside surface of the barrel sidewall. In addition, as illustrated in Fig. 17, cam surface **125** on the stopper is positioned to force sharp free ends **155** further into the syringe barrel wall as more proximally directed force is used in an attempt to improperly reuse the syringe. Accordingly, increased force to pull the plunger rod out of the syringe barrel results in increased force of engagement of the sharp free ends of the locking element into the barrel. It is desirable to provide a cut-out area **126** in the distal end of the plunger rod along the path of the sharp free ends of the locking element for supporting the locking element and allowing it to engage the inside surface of the barrel. Further, the area at the end of the plunger rod on the area around the cutout can be configured to support the locking element so that if the user accidentally withdraws the plunger rod a second time before delivering the final dose of medication the medication may still be delivered even though the locking element sharp free ends are touching the barrel so long as they are moved in a distal direction and urged not to engage the inside surface of the barrel by the cut-out area and the plunger rod. In this case the area around the cutout supports and limits the motion and helps prevent deformation of the sharp free end of the locking element.

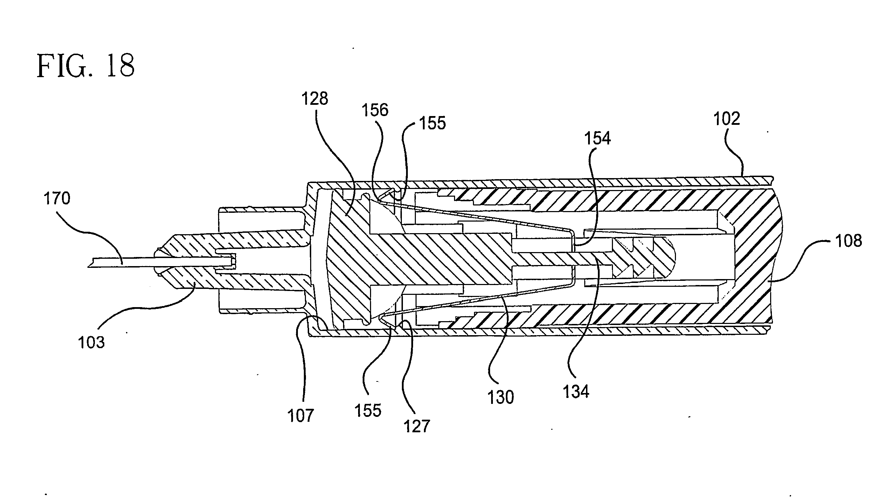

It is also within the purview of the present invention to provide a discontinuity such as a recess or projection on the interior surface of the barrel, as illustrated in Fig. 18, to further improve the engagement of the sharp free end of the locking element with the interior surface of the barrel. In Fig. 18 syringe barrel **102** includes a discontinuity in the form of an inwardly directed projection **127** on inside surface **107** of the barrel. In this embodiment, projection **127** is an annular ring projecting into the barrel and extending 360° around the inside surface. The discontinuity may be in the form of an annular projection, an annular recess or one or more projections or recesses shaped to engage locking element, all positioned within the barrel to engage sharp free end **155** of locking element **130** to further increase the grip of the locking element on the barrel and inside surface.

The present syringe assembly provides an improvement over prior art devices by allowing a variable dose of diluent, chosen by the user at the time of use, to be drawn into the syringe, dispensing the diluent into a vial containing a substance to be reconstituted, drawing a selected amount of the reconstituted substance back into the syringe and then delivering the contents of the syringe. The selected amount of the reconstituted substance may be equal or less than the full volume reconstituted at the discretion of the user. The syringe assembly is automatically disabled after the final injection stroke by reversing the direction of the movement of the plunger rod from the dispensing direction to the aspirating direction. After the injection stroke of the syringe plunger the plunger rod is retracted to activate the disabling mechanism to prevent axial movement of the stopper toward the proximal end of the syringe barrel thereby preventing the stopper from being removed and preventing reuse of the syringe.

When the present syringe assembly has two or more detents on the stopper and in the plunger rod, the maximum number of strokes the syringe assembly will allow can be varied by the initial position of the locking element with respect to the stopper detents and the plunger rod detents.

While various embodiments have been chosen to illustrate the invention, it will be appreciated that changes and modifications can be made without departing from the scope of the invention.

## Claims

1. An operable syringe assembly having passive disabling structure comprising:
a barrel (102) including a cylindrical side wall (106) having an inside surface (107) defining a chamber (109) for retaining fluid, an open proximal end (113) and a distal end (115) including a distal wall (117) having a passageway (119) therethrough in fluid communication with said chamber;
an elongate hollow plunger rod (108) having a proximal end (111), an open distal end (110) and an interior surface (116),
a stopper (128) including a circular shaped sealing element (144) having a peripheral surface (145) forming a seal with said inside surface of said barrel, a boss member (134) projecting proximally from said sealing element,
at least one recess (114) is provided in said interior surface at said distal end of said plunger rod, said recess having a distal face (121), at least one detent (118) on said interior surface at said distal end of said plunger rod,
said stopper comprises at least one boss detention (136) on said boss, at least one cantilevered arm (140) extending proximally from said sealing element (144)
a locking element (130) is provided including a central body portion (148) having an aperture (152) therethrough, at least one cantilevered leg (150) extending distally outwardly from said body portion, and at least one finger element (154) extending proximally inwardly from said aperture (152), said at least one leg (150) having a sharp free end (155) directed outwardly for engaging said inside surface of said barrel,
said locking element (130) being positioned with said sharp free end (155) contacting said interior surface (116) of said plunger rod proximally of said at least one detent, said boss member (134) being positioned in said aperture (152) of said locking element wherein said at least one finger element (154) is contacting said boss proximally of said at least one boss detent (136),
**characterized in that** said at least one cantilevered arm having an outwardly extending rib (142) near its free end, said rib being sized to fit within said recess (114) in said plunger rod (108) wherein said outwardly extending rib (142) being positioned in said recess (114) of said plunger rod (108), so that applying a proximally directed force to said plunger rod while holding said barrel causes said plunger rod to move proximally with respect to said stopper until said free end of said cantilevered leg (150) moves distally along said inner surface of said plunger rod to said at least one detent (118) and said outwardly extending rib (142) on said cantilevered arm engages said distal face (121) of said recess (114) to move said stopper in a proximal direction for a selected distance, and subsequently applying a distally directed force to said plunger rod (108) to discharge fluid from said chamber through said passageway causes said plunger rod to move in a distal direction along with said locking element (130) due to its engagement with said at least one detent until said finger element of said locking element rides over said at least one boss detent (136) and into contact with said at least one boss detent and said plunger rod contacts said stopper (128) to move said stopper distally to discharge fluid from said chamber, after which applying a proximally directed force to said plunger rod will cause said plunger rod to move proximally until said free end (155) of said cantilevered leg (150) moves distally along said inner surface past said distal end (110) of said plunger rod (108) so that said sharp end (155) of said at least one cantilever leg (150) engage said inside of said barrel to help prevent proximal movement of said stopper to render said syringe assembly unusable.

2. The syringe assembly of claim 1 wherein said at least one detent (118) in said plunger rod (108) includes two axially spaced detents (120) and said at least one detent (136) on said boss (134) includes two axially spaced boss detents (136) so that said plunger rod can be moved distally two times before proximal motion of said plunger rod (108) causes said locking element (130) to engage said inside surface of said barrel.

3. The syringe assembly of claim 2 wherein said two axially spaced detents (118) in said plunger rod include two axially spaced steps (120) each having a blunt surface (122) at its distal end extending inwardly from said interior surface.

4. The syringe assembly of claim 2 wherein said two axially spaced boss detents (136) each include an inclined surface (137) extending proximally inwardly and a blunt surface (128) at a distal end of each of said inclined surfaces extending radially inwardly.

5. The syringe assembly of claim 2 wherein said at least one cantilevered leg (150) of said locking element (130) includes two cantilevered legs positioned on opposite sides of said central body portion (148) and two additional axially spaced detents are provided in said plunger rod opposite said axially-spaced detents (118).

6. The syringe assembly of claim 5 further including two radial projections (125) on said stopper (128) positioned to engage and force said two cantilevered legs (150) outwardly when excessive proximally directed force is applied to said plunger rod (108) in an attempt to overcome said locking element's engagement of said inside surface of said barrel.

7. The syringe assembly of claim 1 wherein said at least one cantilevered arm (150) of said stopper includes two cantilevered arms positioned on opposite sides of said boss (134) and said at least one recess (114) in said inner surface of said plunger rod (108) includes two recesses positioned on opposite sides of said plunger rod and positioned to receive said outward extending ribs (142) of said cantilevered arms (140).

8. The syringe assembly of claim 1 having a discontinuity (127) on said inside surface of said barrel sidewall positioned to engage said sharp free end (155) of said locking element (130) when said sharp free end is contacting said inside surface of said barrel.

9. The syringe assembly of claim 1 wherein said distal wall of said barrel further includes an elongate tip (103) extending distally therefrom having a passageway in fluid communication with said passageway in said distal wall (117).

10. The syringe assembly of claim 1 further including a needle cannula (170) having a distal end, a proximal end and a lumen therethrough, said proximal end of said needle cannula being connected to said distal end (115) of said barrel so that said lumen is in fluid communication with said passageway.

11. The syringe of claim 1 wherein said locking element (130) is made of sheet metal.

12. The syringe assembly of claim 1 wherein said stopper (138) is integrally formed of thermoplastic material.

## Patentansprüche

1. Betätigbare Spritzenanordnung mit einer passiven Deaktivierungsstruktur, mit:
einem Zylinder (102) mit einer zylindrischen Seitenwand (106), welche eine Innenfläche (107) aufweist, die eine Fluidaufnahmekammer (109) bildet, einem offenen proximalen Ende (113) und einem distalen Ende (115), das eine distale Wand (117) mit einem hindurchgehenden Durchlass (119) aufweist, welcher in Fluidverbindung mit der Kammer steht;
einer langgestreckten hohlen Kolbenstange (108) mit einem proximalen Ende (111), einem offenen distalen Ende (110) und einer Innenfläche (116);
einem Stopfen (128) mit einem kreisförmigen Dichtelement (144), das eine Umfangsfläche (145) aufweist, welche eine Dichtung mit der Innenfläche des Zylinders bildet, wobei ein Ansatzelement (134) proximal von dem Dichtelement absteht,
wobei mindestens eine Ausnehmung (114) in der Innenfläche am distalen Ende der Kolbenstange vorgesehen ist, wobei die Ausnehmung eine distale Fläche (121), wobei mindestens eine Raste (118) auf der Innenfläche am distalen Ende der Kolbenstange ausgebildet ist,
wobei der Stopfen mindestens eine Ansatzraste (136) an dem Ansatz aufweist, wobei sich mindestens ein freitragender Arm (140) von dem Dichtelement (144) in proximaler Richtung erstreckt,
wobei ein Verriegelungselement (130) vorgesehen ist, welches einen mittleren Körperbereich (148) mit einer hindurchgehenden Öffnung (152), mindestens einen freitragenden Schenkel (150), der sich von dem Körperbereich distal nach außen erstreckt, und mindestens ein Fingerelement (154) aufweist, das sich von der Öffnung (152) proximal nach innen erstreckt, wobei der mindestens eine Schenkel (150) ein nach außen gerichtetes spitzes freies Ende (155) aufweist, das zum Angreifen an der Innenfläche des Zylinders nach außen gerichtet ist;
wobei das Verriegelungselement (130) derart angeordnet ist, dass das spitze freie Ende (155) die Innenfläche (116) der Kolbenstange proximal der mindestens einen Raste berührt, wobei das Ansatzelement (134) in der Öffnung (152) des Verriegelungselements angeordnet ist, wobei das mindestens eine Fingerelement den Ansatz proximal der mindestens einen Pfostenraste (136) berührt,
**dadurch gekennzeichnet, dass**
der mindestens eine freitragende Arm eine sich nach außen erstreckende Rippe (142) nahe seinem freien Ende aufweist, wobei die Rippe derart bemessen ist, dass sie in die Ausnehmung (114) in der Kolbenstange (108) passt;
wobei die sich nach außen erstreckende Rippe (142) in der Ausnehmung (114) der Kolbenstange (108) derart angeordnet ist, dass das Aufbringen einer proximal gerichteten Kraft auf die Kolbenstange, während der Zylinder gehalten wird, bewirkt, dass sich die Kolbenstange in Bezug auf den Stopfen in proximaler Richtung bewegt, bis das freie Ende des freitragenden Schenkels (150) sich distal entlang der Innenfläche der Kolbenstange zu der mindestens einen Raste (118) bewegt und die sich auswärts erstreckende Rippe (142) an dem freitragenden Arm an der distalen Fläche (121) der Ausnehmung (114) angreift, um den Stopfen über eine gewählte Entfernung in proximaler Richtung zu bewegen, und wobei das anschließende Aufbringen einer distal gerichteten Kraft auf die Kolbenstange (108) zum Ausgeben von Fluid aus der Kammer durch den Durchlass bewirkt, dass sich die Kolbenstange aufgrund ihres Zusammengreifens mit der mindestens einen Raste zusammen mit dem Verriegelungselement (130) in distaler Richtung bewegt, bis das Fingerelement des Verriegelungselements sich über die mindestens eine Ansatzraste (136) bewegt und in Kontakt mit der mindestens einen Ansatzraste gelangt und die Kolbenstange den Stopfen (128) berührt, um den Stopfen zur Ausgabe von Fluid aus der Kammer distal zu bewegen, wonach das Aufbringen von proximal gerichteter Kraft auf die Kolbenstange bewirkt, dass sich die Kolbenstange proximal bewegt, bis sich das freie Ende (155) des freitragenden Schenkels (150) distal entlang der Innenfläche an dem distalen Ende (110) der Kolbenstange (108) vorbei bewegt, so dass das spitze Ende (155) des mindestens einen freitragenden Schenkels (150) an der Innenfläche des Zylinders angreift, um das Verhindern der proximalen Bewegung des Stopfens zu unterstützen und die Spritzenanordnung unbrauchbar zu machen.

2. Spritzenanordnung nach Anspruch 1, bei welcher die mindestens eine Raste (118) in der Kolbenstange (108) zwei axial beabstandete Rasten (120) umfasst und die mindestens eine Raste (136) an dem Ansatz (134) zwei axial beabstandete Ansatzrasten (136) umfasst, so dass die Kolbenstange zwei Mal distal bewegt werden kann, bevor die proximale Bewegung der Kolbenstange (108) das Angreifen des Verriegelungselements (130) an der Innenfläche des Zylinders bewirkt.

3. Spritzenanordnung nach Anspruch 2, bei welcher die beiden axial beabstandeten Rasten (118) in der Kolbenstange zwei axial beabstandete Stufen (120) aufweisen, die jeweils eine stumpfe Oberfläche (122) an ihrem distalen Ende aufweisen, welche sich von der Innenfläche nach innen erstreckt.

4. Spritzenanordnung nach Anspruch 2, bei welcher die beiden axial voneinander beabstandeten Ansatzrasten (136) jeweils eine sich proximal nach innen erstreckende Schrägfläche (137) und eine radial nach innen gerichtete stumpfe Fläche (138) an einem distalen Ende jeder der Schrägflächen aufweisen.

5. Spritzenanordnung nach Anspruch 2, bei welcher der mindestens eine freitragende Schenkel (150) des Verriegelungselements (130) zwei freitragende Schenkel, die auf gegenüberliegenden Seiten des mittleren Körperbereichs (148) angeordnet sind, aufweist, und zwei zusätzliche axial beabstandete Rasten in der Kolbenstange den axial beabstandeten Rasten (118) gegenüberliegend vorgesehen sind.

6. Spritzenanordnung nach Anspruch 5, ferner mit zwei radialen Vorsprüngen (125) an dem Stopfen (128), die derart angeordnet sind, dass sie an den beiden freitragenden Schenkeln (150) angreifen und diese nach außen drücken, wenn eine übermäßige proximal gerichtete Kraft auf die Kolbenstange (108) bei einem Versuch aufgebracht wird, den Eingriff des Verriegelungselements an der Innenfläche des Zylinders zu überwinden.

7. Spritzenanordnung nach Anspruch 1, bei welcher der mindestens eine freitragende Arm (140) des Stopfens zwei freitragende Arme aufweist, welche auf gegenüberliegenden Seiten des Ansatzes (134) angeordnet sind, und die mindestens eine Ausnehmung (114) in der Innenfläche der Kolbenstange (108) zwei Ausnehmungen aufweist, welche auf gegenüberliegenden Seiten der Kolbenstange angeordnet sind und zum Aufnehmen der sich nach außen erstreckenden Rippen (142) der freitragenden Arme (140) angeordnet sind.

8. Spritzenanordnung nach Anspruch 1 mit einer Diskontinuität (127) auf der Innenfläche der Zylinderseitenwand, welche derart angeordnet ist, dass sie mit dem spitzen freien Ende (155) des Verriegelungselements (130) zusammengreift, wenn das spitze freie Ende die Innenfläche des Zylinders berührt.

9. Spritzenanordnung nach Anspruch 1, bei welcher die distale Wand des Zylinders ferner eine langgestreckte Spitze (103) aufweist, die sich distal von dieser erstreckt und einen Durchlass aufweist, welcher in Fluidverbindung mit dem Durchlass in der distalen Wand (117) steht.

10. Spritzenanordnung nach Anspruch 1, ferner mit einer Nadelkanüle (170) mit einem distalen Ende, einem proximalen Ende und einem durchgehenden Lumen, wobei das proximale Ende der Nadelkanüle mit dem distalen Ende (115) des Zylinders verbunden ist, so dass das Lumen in Fluidverbindung mit dem Durchlass steht.

11. Spritzenanordnung nach Anspruch 1, bei welcher das Verriegelungselement (130) aus Blech besteht.

12. Spritzenanordnung nach Anspruch 1, bei welcher der Stopfen (128) einstückig aus thermoplastischem Material gebildet ist.

## Revendications

1. Ensemble seringue actionnable ayant une structure de désactivation passive, comprenant:
un cylindre (102) comprenant une paroi latérale (106) ayant une face interne (107) définissant une chambre (109) pour contenir de fluide, une extrémité proximale ouverte (113) et une extrémité distale (115) incluant une paroi distale (117) ayant un passage (119) à travers celle-ci, qui est en communication fluidique avec ladite chambre;
une tige de piston (108) creux élongée avec une extrémité proximale (111). Une extrémité distale ouverte (110) et un face intérieure (116),
un bouchon (128) comprenant un élément d'étanchéité (144) circulaire avec une face périphérique (145) formant un joint avec la face interne du cylindre, un pilier (134) s'étendant proximalement à partir dudit élément d'étanchéité,
au moins un creux (114) étant prévue dans la face intérieure à l'extrémité distale de ladite tige de piston, ledit creux comprenant une face distale (121), au moins une encoche (118) étant ménagée sur ladite face intérieure à l'extrémité distale de ladite tige de piston,
ledit bouchon comprenant au moins une encoche de pilier (136) sur ledit pilier, au moins un bras à porte à faux (140) s'étendant proximalement à partir dudit élément d'étanchéité (144),
un élément de verrouillage (130) ayant une partie de corps centrale (148) avec une aperture (152) à travers celle-ci, au moins une branche à porte à faux (150) s'étendant distalement vers l'extérieur à partir de ladite partie de corps, et au moins un élément de doigt (154) s'étendant proximalement vers l'intérieur dans ladite aperture (152), ladite au moins une branche (150) ayant une extrémité libre pointée (155) orientée vers l'extérieur pour engager la face intérieure dudit cylindre;
ledit élément de verrouillage (130) étant positionnée avec ladite extrémité libre pointée (155) en contact avec la face intérieure (116) de ladite tige de piston proximalement de ladite au moins une encoche, ledit pilier (134) étant positionné dans ladite aperture (152) dudit élément de verrouillage, ledit au moins élément de doigt (154) étant en contact avec ledit pilier proximal de ladite au moins une encoche de pilier (136),
**caractérisé en ce que**
ledit au moins un bras à porte à faux (140) comprend une nervure (142) à proximité de son extrémité libre, ladite nervure étant dimensionnée pour entrer dans le creux (114) dans ladite tige de piston (108), ladite nervure (142) orientée vers l'extérieur étant positionnée dans ledit creux (114) de ladite tige de piston (108) de manière que l'application de force dans le sens proximal sur ladite tige de piston, tout en tenant ledit cylindre, provoque le mouvement proximal de la tige de piston par rapport audit bouchon jusqu'à ce que ladite extrémité libre de ladite branche à porte à faux (150) se déplace distalement le long ladite face intérieure de la tige de piston vers ladite au moins une encoche (118) et ladite nervure (142) orientée vers l'extérieur sur ledit bras à porte à faux engage ladite face distale (121) dudit creux (114) pour déplacer ledit bouchon dans la direction proximale, pour une distance choisie, et l'application suivante d'une force orientée distalement sur ladite tige de piston (108) pour distribuer de la fluide de ladite chambre à travers ledit passage provoque le déplacement de ladite tige de piston dans la direction distale conjointement avec ledit élément de verrouillage (130) à cause de son engagement avec ladite au moins une encoche, jusqu'à ce que ledit élément de doigt dudit élément de verrouillage passe au-dessus de ladite au moins une encoche de pilier (136) et en contact avec ladite au moins une encoche de pilier et ladite tige de piston s'appui contre ledit bouchon (128) pour déplacer ledit bouchon distalement pour distribuer de la fluide de ladite chambre, ensuite de quoi l'application d'une force orientée proximalement sur ladite tige de piston cause le déplacement de ladite tige de piston dans la direction proximale, jusqu'à ce que ladite extrémité libre (155) de la branche (150) à porte à faux se déplace distalement le long de la face interne dudit creux, passant ladite extrémité (110) distale de ladite tige de piston (108) de sorte que ladite extrémité pointée (155) de ladite au moins une branche à porte à faux (150) vient en prise avec ladite face intérieure dudit cylindre pour assister à empêcher le déplacement proximal dudit bouchon afin de rendre ledit ensemble de seringue inutilisable.

2. Ensemble seringue selon la revendication 1, dans lequel ladite au moins une encoche (118) dans ladite tige de piston (108) comprend deux encoches (120) écartées axialement et l'au moins une encoche (136) sur ledit pilier (134) comprend deux encoches de pilier (136) écartées axialement, de sorte que ladite tige de piston peut être déplacée distalement deux fois avant le déplacement proximal de ladite tige de piston (108) fait ledit élément de verrouillage (130) engager ladite face interne dudit cylindre.

3. Ensemble seringue selon la revendication 2, dans lequel les deux encoches (118) dans ladite tige de piston, écartées axialement, comprennent deux gradins (120) écartés axialement, chaque gradin ayant une face émoussée (122) à son extrémité distale s'étendant vers l'intérieur à partir de la face interne.

4. Ensemble seringue selon la revendication 2, dans lequel chacune desdites deux encoches de pilier (136) écartées axialement comprend une face inclinée (137) s'étendant proximalement vers l'intérieur et une face émoussée (138) s'étendant radialement et ménagée à l'extrémité distale de chaque face inclinée.

5. Ensemble seringue selon la revendication 2, dans lequel ladite au moins une branche (150) à porte à faux dudit élément de verrouillage (130) comprend deux branches à porte à faux positionnées sur deux côtés opposés de la partie de corps centrale (148), et deux encoches additionnelles écartées axialement sont prévues dans ladite tige de piston opposé lesdites encoches (118) écartées axialement.

6. Ensemble seringue selon la revendication 5, comprenant en outre deux saillies radiales (125) sur ledit bouchon (128), positionnées pour engager les deux branches (150) à porte à faux et forcer celles-ci vers l'extérieur quand une forces proximale excessive est appliquée sur ladite tige de piston (108) lors d'un essai de franchir la prise dudit élément de verrouillage avec la face interne du cylindre.

7. Ensemble seringue selon la revendication 1, dans lequel ledit au moins un bras à porte à faux (140) dudit bouchon comprend deux bras á porte à faux positionnés sur des côtés opposés dudit pilier (134), et ledit au moins un creux (114) dans la face interne de ladite tige de piston (108) comprend deux creux positionnés sur des côtés opposés de ladite tige de piston et positionnés pour recevoir les nervures (142) des bras à porte à faux (140), orientées vers l'extérieur.

8. Ensemble seringue selon la revendication 1, comprenant une discontinuité (127) sur la face interne de la paroi latérale du cylindre, positionnée pour engager l'extrémité libre pointée (155) dudit élément de verrouillage (130) quand ladite extrémité libre pointée est en contact avec la face interne dudit cylindre.

9. Ensemble seringue selon la revendication 1, dans lequel la paroi distale du cylindre comprend en outre une pointe élongée (103) s'étendant distalement á partir de celle-ci et comprenant un passage en communication fluidique avec le passage dans ladite paroi distale (117).

10. Ensemble seringue selon la revendication 1, comprenant en outre une aiguille de canule (170) avec une extrémité distale, une extrémité proximale et une lumière à travers ladite aiguille, ladite extrémité proximale de ladite aiguille de canule étant raccordée à l'extrémité distale (115) dudit cylindre de manière que ladite lumière est en communication fluidique avec ledit passage.

11. Ensemble seringue selon la revendication 1, dans lequel ledit élément de verrouillage (130) est en tôle de métal.

12. Ensemble seringue selon la revendication 1, dans lequel ledit bouchon (128) est formé en une pièce d'un matériau thermoplastique.
